Europäisches Patentamt

European Patent Office

Office européen des brevets

(11) Veröffentlichungsnummer: 0 214 353
A1

# (12) EUROPÄISCHE PATENTANMELDUNG

(21) Anmeldenummer: 85810419.3

(22) Anmeldetag: 13.09.85

(51) Int. Cl.⁴ A61N 5/06

(43) Veröffentlichungstag der Anmeldung:
18.03.87 Patentblatt 87/12

(84) Benannte Vertragsstaaten:
AT BE CH DE FR GB IT LI LU NL SE

(71) Anmelder: Wolff, Friedrich
Störklingasse 38
CH-4125 Riehen/Basel(CH)

(72) Erfinder: Wolff, Friedrich
Störklingasse 38
CH-4125 Riehen/Basel(CH)

(74) Vertreter: Eschmann, Heinz et al
A. Braun, Braun, Héritier, Eschmann AG
Patentanwälte Holbeinstrasse 36-38
CH-4051 Basel(CH)

(54) UV-Bestrahlungsgerät.

(57) Das Bestrahlungsgerät weist ein Kunststoffgehäuse (1) auf, in welchem eine Reihe von Reflektoren (6) mit UV-Röhren (5) unter einer Acrylglasplatte (7) angeordnet sind. Das Gehäuse (1) ist mit Füssen (2, 3) versehen, deren einer (3) Rollen (4) zum bequemen Transport aufweist.

Auf die Acrylglasplatte (7) ist eine gasdichte Hülle (8) aufgeschweisst, die sich nach Art einer Luftmatratze aufpumpen lässt. Die mehrfach durch Schweissnähte unterteilte, aufgeblasene Hülle (8) bietet eine bequeme Liegefläche mit geringen Transmissionsverlusten. Eine auf dem Bauche liegende Person kann auch im Gesichtsbereiche direkt auf der Hülle (8) aufliegen, so dass der Vorteil der bequemen Lage durch eine hohe Strahlendichte ergänzt wird.

FIG. 4

EP 0 214 353 A1

## UV-Bestrahlungsgerät

Die vorliegende Erfindung betrifft ein Gerät zur UV-Bestrahlung, insbesondere Bräunung, des menschlichen Körpers, mit einem Gehäuse, in welchem eine Reihe von Bestrahlungsröhen und deren Reflektoren untergebracht sind.

Derartige, bekannte Geräte sind z.B. als sogenannte Sonnenbänke ausgebildet, auf welchen die zu bestrahlende Person zur Erzielung der erwünschten Ganzkörperbestrahlung abwechselnd auf dem Rücken und auf dem Bauche liegt. Im letzteren Falle kann der Kopf des Liegenden nicht auf die harte Acrylglasplatte gelegt werden und wird daher meist im Abstand von derselben gehalten, was auf die Dauer unbequem ist und zu Verspannungen führt. Auch ist dadurch die Bestrahlungsstärke im Gesichtsbereich in Anbetracht der mit dem Quadrat der Entfernung von der Strahlenquelle abnehmenden Strahlungsdichte relativ gering.

Die gleichen Nachteile gelten sinngemäss auch für die Teilkörper-Bestrahlungsgeräte, die im übrigen an Stativen befestigt und daher unhandlich sind und ferner praktisch nur in sitzender Stellung benutzt werden können, so dass hier infolge der Entfernung des Geräts vom zu bräunenden Objekt eine relativ geringe Strahlendichte vorliegt und der erwünschte Direktkontakt, bei welchem die zu bestrahlende Körperpartie direkt auf dem Gerät aufliegt, nicht möglich ist.

Um einen direkten Kontakt des Körpers mit dem Bestrahlungsgerät auch im Gesichtsbereich zu erzielen, könnte man daran denken, auf die Acrylglasplatte eine herkömmliche Luftmatratze oder ein sonstiges Polster aufzulegen. Diese Lösung kommt aber aufgrund der damit verbundenen Transmissionsverluste nicht in Frage.

Es ist somit die Aufgabe der vorliegenden Erfindung, ein Gerät der vorerwähnten Art vorzuschlagen, das ein direktes Auflegen der zu bestrahlenden Körperpartie, insbesondere des Gesichts, auf das Gerät ermöglicht und dabei einerseits eine grösstmögliche Strahlendichte im Hauptbereich erzielt und andererseits unter Vermeidung von Ermüdungs-und Verspannungserscheinungen ein bequemes Liegen während der gesamten Bestrahlungsdauer zulässt.

Gemäss einem weiteren Aspekt der Erfindung soll das erfindungsgemässe Gerät wenig Raum einnehmen, leicht transportierbar und auch auf geringem Raum versorgbar sein.

Die Erfindung ist im unabhängigen Patentanspruch 1 definiert. Ausführungsvarianten ergeben sich aus den abhängigen Patentansprüchen.

Nachstehend wird anhand der beiliegenden Zeichnung ein Ausführungsbeispiel des Erfindungsgegenstandes mit einigen konstruktiven Varianten erläutert. Es zeigen:

Fig. 1 eine Perspektivansicht einer ersten Ausführungsform der Erfindung,

Fig. 2 das Gerät in vertikaler gebrauchslage, die auch als platzsparende Versorgungslage dient,

Fig. 3 die horizontale Gebrauchslage,

Fig. 4 eine teilweise aufgeschnittene perspektivische Teildarstellung des gleichen Gerätes,

Fig. 5 eine vereinfachte Draufsicht,

Fig. 6 eine perspektivische Schnittdarstellung im Kopfbereich des Gerätes,

Fig. 7 eine teilweise aufgeschnittene Perspektive einer zweiten Ausführungsform,

Fig. 8 eine perspektivische Gesamtansicht des in Fig. 6 dargestellten Gerätes.

Fig. 9 eine Perspektivansicht einer Ausführungsvariante des in Fig. 8 gezeigten Beispiels,

Fig. 10 einen Vertikalschnitt dieser Ausführungsvariante,

Fig. 11 die zugehörige Draufsicht und

Fig. 12 eine weitere Möglichkeit zur gasdichten, lösbaren Befestigung der Hülle.

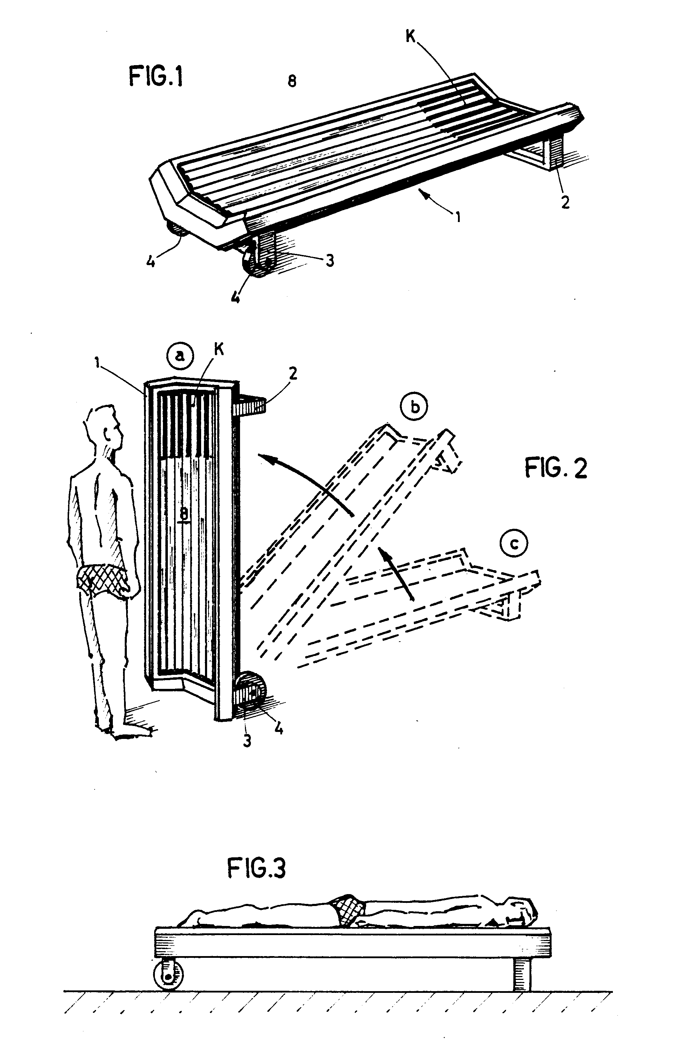

Das in Fig. 1 unter Verzicht auf unwesentliche Einzelheiten dargestellte Gerät weist ein langgestrecktes Kunststoffgehäuse 1 auf, das in Bankform ausgebildet und demgemäss im Bereich jeder Stirnseite mit einem Fuss 2 bzw. 3 versehen ist. Am Fuss 3 befinden sich zwei rollen 4, so dass das Gerät aus seiner vertikalen Gebrauchslage (a) in eine Transportlage (b) umgekippt und in dieser wie eine Schubkarre transportiert werden kann, worauf es je nach Bedarf in die horizontale Gebrauchslage (c) abgestellt wird. Auf diese Weise lässt sich das gerät, das immerhin bis zu ...... kg wiegen kann, leicht bewegen und auch auf kleinstem Raume versorgen.

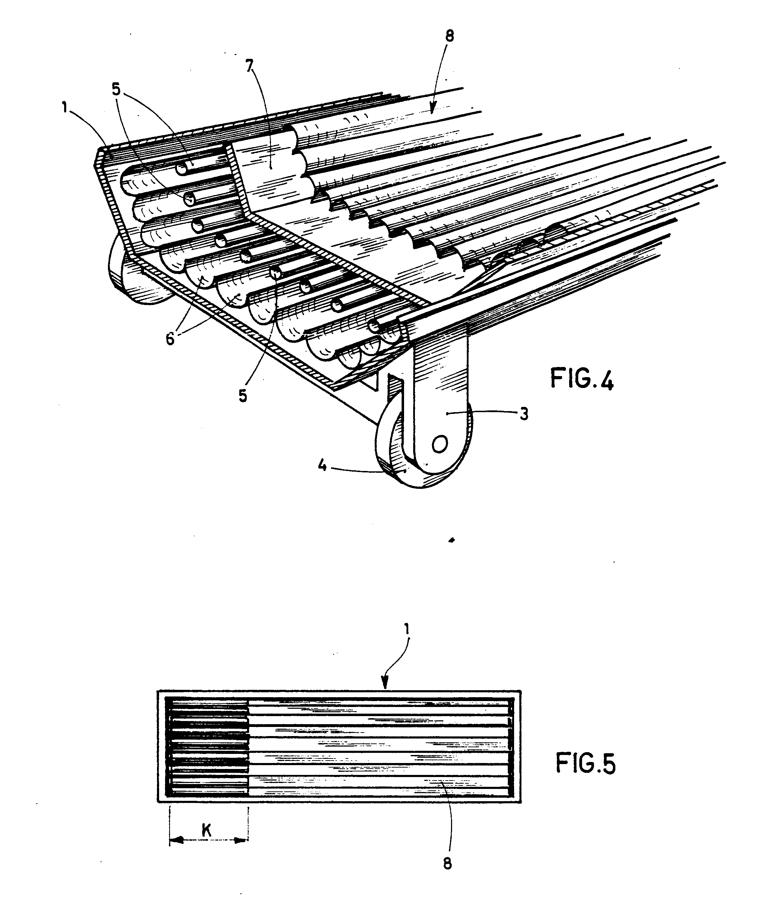

Gemäss Fig. 4 sind innerhalb des Gehäuses 1 eine Reihe von UV-Röhren 5 befestigt, denen ebensoviele Reflektoren 6 zugeordnet sind. Oberhalb der UV-Röhren befindet sich eine ebene oder auch konkav gewölbte, UV-durchlässige Acrylglasplatte 7, die das Gehäuse 1 nach oben abschliesst und als Teil des Gehäuses angesehen werden kann.

Um nun den direkten Kontakt des zu bestrahlenden Körpers auch im Gesichtsbereich mit dem Gerät und damit eine intensive Bestrahlung in bequemer Lage zu ermöglichen, ist auf der Acrylglasplatte 7 eine biegsame Hülle 8 aus einem UV-Strahlen-durchlässigen und gasdichten Material befestigt. Um eine allseitige Gasdichtheit zu erzielen, ist die Hülle 8 mit dem Acrylglas im gesamten Randbereich der Platte 7 verbunden; andererseits ist die Hülle 8 nach Art einer Luftmatratze durch weitere längs-oder querverlaufende Schweissnähte S bis auf einige Luftdurchtritte unterteilt und mit einem nicht dargestellten Aufpumpventil versehen. Die der zu bestrahlenden Person zugewandte Seite der Acrylglasplatte 7 bietet somit eine bequeme Abstützfläche, auf die unter anderem auch das Gesicht während der Bestrahlungsdauer direkt aufgelegt werden kann. In dieser bequemen Lage ist die Distanz zur Strahlungsquelle so gering, dass sich unter weiterer Berücksichtigung der geringen Transmissionsverluste der einlagigen Hülle 8 im Hautbereich eine hohe Strahlungsdichte ergibt.

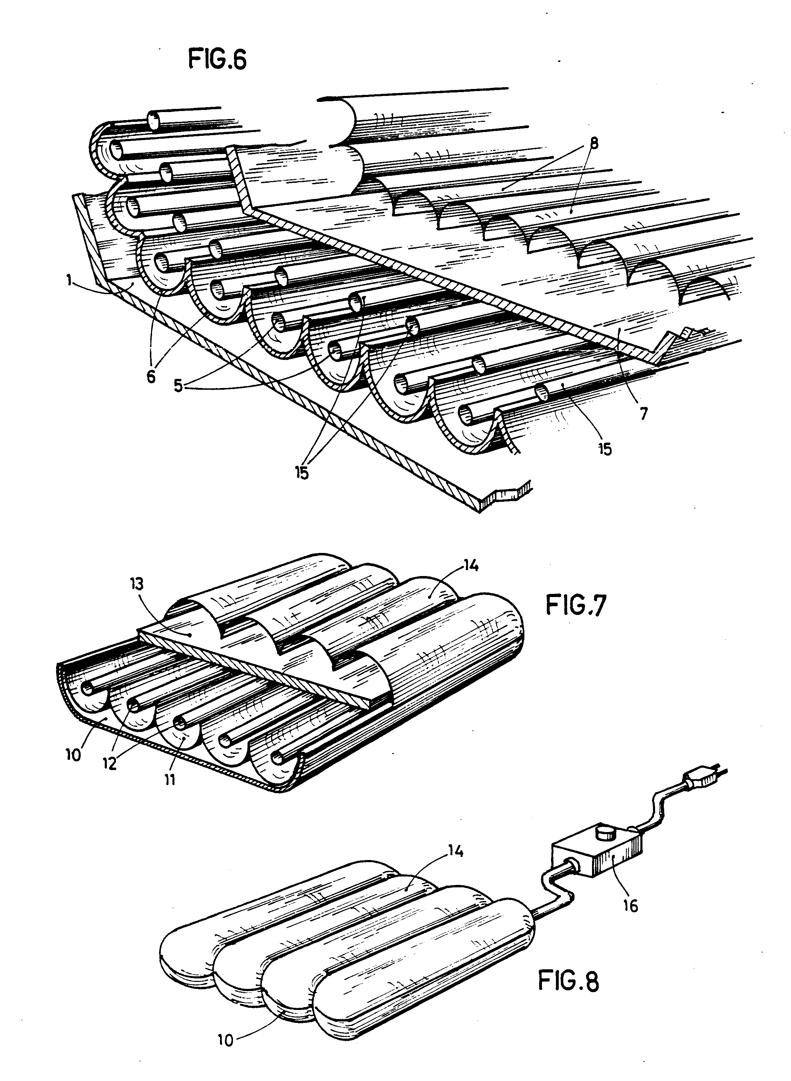

Wie die Fig. 5 und 6 ferner zeigen, können gemäss einer weiteren Ausführungsform im Kopfbereich K zwischen der Acrylglasplatte 7 und den verkürzten Kontaktzonen der Reflektoren 6 mehrere kleinere UV-Röhren 15 zur Gesichtsbräunung zusätzlich angeordnet sein.

Gemäss einer weiteren, in Fig. 7 und 8 dargestellten Variante der Erfindung ist das erfindungsgemässe Gerät in Form eines flachen Kissens 9 ausgebildet, das ein Kunststoffgehäuse 10, mehrere flache Reflektoren 11, mehrere UV-Röhren 12 kleinen Durchmessers, eine Acrylglasplatte 13 und eine aufblasbare Hülle 14 aufweist.

Dieses flache, zur Teilkörper-und insbesondere Gesichtsbestrahlung gedachte Gerät, dessen Vorschaltgerät 16 separat vorgesehen ist, ist im Gegensatz zu den bekannten Teilkörperbestrahlern äusserst handlich und in jedem Reisekoffer verstaubar und gestattet es vor. allem, dass auch das Gesicht in bequemer Lage wie auf ein Kissen direkt aufgelegt werden kann.

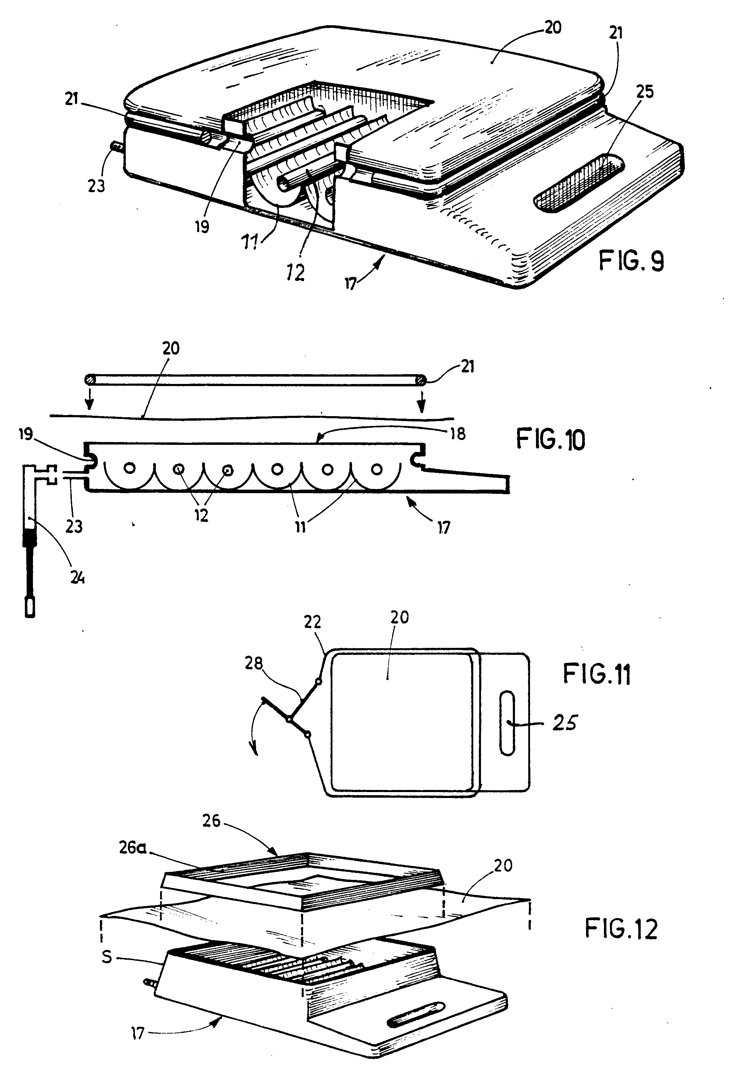

Eine Variante zu der in Fig. 8 gezeigten Ausführungsform zeigen die Fig. 9 bis 11. Ein Gehäuse 17 ist wiederum mit Reflektoren 11 und UV-Röhren 12 versehen. Oberhalb der UV-Röhren 12 ist das Gehäuse 17 offen, d.h., dass die mit 18 bezeichnete Oeffnung nicht durch eine Acrylglasplatte abgedeckt ist. Die Seitenwände des Gehäuses 17 weisen in ihrem oberen Teil eine ringsumlaufende Nut 19 auf; eine biegsame, gasdichte Hülle 20 lässt sich somit über die Gehäuseöffnung 18 legen und mit ihrem Randbereich durch einen Spannring 21 in der Nut 19 verankern. Der Spannring 21 kann ein einfacher, der Querschnittsform der Nut 19 angepasster Gummiring sein. Er kann aber auch, wie in Fig. 11 gezeigt, als unelastischer Gurt 22 mit einem Kniehebelverschluss 23 ausgebildet sein.

Die Hülle 20 sollte jedenfalls gasdicht das Gehäuse 17 überspannen, wobei die Seitenwände des Ge häuses 17 mit dessen Boden eine gasdichte Einheit bilden. Mittels eines am Gehäuse 17 angeordneten Ventils 23 und einer Handpumpe 24 lässt sich das gasdichte Gehäuse 17 aufpumpen, so dass die pneumatisch abgestützte Hülle 20 eine weiche Liegerfläche für den zu bestrahlenden Körperteil bildet.

In der Gehäuseöffnung 18 kann aus Festigkeitsgründen ein Stützgitter angebracht sein.

Wie die Fig. 9 bis 11 ferner zeigen, kann das Gehäuse 17 an einer Seite etwas abgesetzt und mit einem Handgriff 25 versehen sein, der das Tragen des kissenförmigen Gerätes erleichtert.

Die gasdichte, vorzugsweise lösbare Verankerung der Hülle 20 am Gehäuse 17 kann vom Fachmann auf vielerlei Weise bewerkstelligt werden. Gemäss Fig. 12 sind die Seitenwände S des Gehäuses nach innen geneigt. Die Hülle 20 wird durch einen dieser Neigung angepassten Klemmring 26 zwischen dessen geneigter Klemmfläche 26a und der Aussenseite der Seitenwände S eingeklemmt und ist durch Klemmwirkung gasdicht verankert.

Die beschriebenen Ausführungsbeispiele können vom Fachmann in mehrfacher Hinsicht im Rahmen des Erfindungsgedankens abgewandelt werden. Die gasdichte Hülle soll UV-durchlässig und UV-beständig, falls erforderlich mit UV-durchlässigem Acrylglas verschweissbar sowie mechanisch widerstandsfähig und hautfreundlich sein. Beigsame Hüllen aus Polyvinylfluorid oder Hochdruck-Polyaethylen sind für diesen Zweck bestens geeignet.

**Ansprüche**

1. Gerät zur UV-Bestrahlung, insbesondere Bräunung, des menschlichen Körpers, mit einem Gehäuse (1), in welchem eine Reihe von Bestrahlungsröhren (5, 12) und deren Reflektoren (6, 10) untergebracht sind, dadurch gekennzeichnet, dass diejenige Seite des Gehäuses (7, 13), die als Liegefläche für den zu bestrahlenden Körper bzw. Körperteil gedacht ist, mindestens teilweise von einer biegsamen, gasundurchlässigen Hülle (8, 14) überdeckt ist, welche gasdicht mit dem darunter befindlichen Gehäuse (7, 13) verbunden ist, wobei ein Druckluft-Anschlussorgan vorgesehen ist, so dass sich der unterhalb der gasundurchlässigen Hülle (8, 14) befindliche Raum nach Art einer Luftmatratze aufblasen lässt.

2. Gerät nach Anspruch 1, wobei das Gehäuse (1) auf der Bestrahlungsseite durch eine UV-durchlässige Acrylglasplatte abgedeckt ist, dadurch gekennzeichnet, dass die biegsame, gasundurchlässige Hülle (8, 14) auf der Aussenseite der Acrylglasplatte (7, 13) angeordnet und mit der Acrylglasplatte gasdicht verbunden ist.

3. Gerät nach Anspruch 2, zur Ganzkörper-einschliesslich Gesichtsbestrahlung, mit einem der Grösse des menschlichen Körpers angepassten Gehäuse (1), das als Ruhebank ausgebildet und demgemäss im Bereich der beiden Stirnseiten mit Füssen (2, 3) versehen ist, dadurch gekennzeichnet, dass mindestens der zur Abstützung des Kopfes vorgesehene Abschnitt der Acrylglasplatte mit einer angeschweissten, aufpumpbaren gasdichten Hülle (8) versehen ist, so dass auch der Kopf einer auf dem Bauche liegenden Person selbst bei längerer Bestrahlungsdauer entspannt auf der druckluftelastisch abgefederten Hülle (8) aufliegen kann.

4. Gerät nach Anspruch 3, dadurch gekennzeichnet, dass dasselbe im Bereich eines Fusses - (3) mit Rollen (4) versehen ist, so dass das sowohl in vertikaler als auch in horizontaler Lage verwendbare Gerät nach Art einer Schubkarre transportiert und raumsparend versorgt werden kann.

5. Gerät nach einem der Ansprüche 3 oder 4, dadurch gekennzeichnet, dass in dem zur Auflage des Kopfes vorgesehenen Bereich eine Reihe der Gesichtsbräunung dienender Zusatzröhen (15) angeordnet sind, welche jeweils zwischen der Kontaktstelle zweier benachbarter Reflektoren (6) und der darüber befindlichen Acrylglasplatte (7) montiert sind.

6. Gerät nach Anspruch 1, zur Teilkörper-, insbesondere Gesichtsbestrahlung, dadurch gekennzeichnet, dass dasselbe in Form eines flachen Kissens (9) mit separatem Vorschaltgerät (16) ausgebildet ist.

7. Gerät nach Anspruch 6, dadurch gekennzeichnet, dass dasselbe ein gasdichtes Gehäuse - (7) aufweist, dessen obere, bestrahlungsseitige Oeffnung (18) durch die biegsame, gasundurchlässige Hülle (20) abgedeckt ist, die ihrerseits gasdicht mit dem Gehäuse (17) verbunden ist (Fig. 10).

8. Gerät nach Anspruch 7, dadurch gekennzeichnet, dass die genannte Hülle (20) lösbar an den Seitenwänden des Gehäuses (17) befestigt ist.

9. Gerät nach Anspruch 8, dadurch gekennzeichnet, dass das Gehäuse (17) im oberen Teil seiner Seitenwände eine ringsumlaufende Nut (19) aufweist, in der die Hülle (20) mittels eines Spannorganes (23) gasdicht befestigbar ist.

10. Gerät nach Anspruch 8, dadurch gekennzeichnet, dass die Seitenwände (5) des Gehäuses nach innen geneigt sind und ein dieser Neigung angepasster Klemmring (26) vorgesehen ist, um die Hülle (20) zwischen der Aussenwand des Gehäuses (27) und dem Klemmring (26) einzuspannen.

FIG.1

FIG. 2

FIG.3

FIG.4

FIG.5

FIG.6

FIG.7

FIG.8

FIG. 9

FIG. 10

FIG. 11

FIG. 12

**Europäisches Patentamt**

**EUROPÄISCHER RECHERCHENBERICHT**

Nummer der Anmeldung

EP 85 81 0419

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| X | DE-A-2 911 758 (MUTZHAS) * Seite 11, Zeilen 11-19,27-29; Figuren 1,2 * | 1 | A 61 N 5/06 |
| A | | 3,8-10 | |
| | --- | | |
| Y | BE-A- 882 802 (MICHOLT) * Insgesamt * | 1 | |
| A | | 2,3 | |
| | --- | | |
| Y | DE-A-2 601 939 (WOLFF) * Seite 3, Abschnitt 2; Seite 4, Abschnitt 6 * | 1 | |
| A | | 2,3 | RECHERCHIERTE SACHGEBIETE (Int. Cl. 4) |
| | --- | | A 61 N |
| A | US-A-3 170 172 (KESSMAN) * Spalte 1, Zeile 36 - Spalte 2, Zeile 22 * | 1-3 | A 47 C |
| | --- | | |
| A | DE-A-2 603 460 (WOLFF) * Seite 10, Abschnitt 2; Figur 4 * | 4 | |
| | --- | | |
| A | FR-A-2 430 240 (WOLFF) * Seite 1, Zeilen 22-29 * | 6 | |
| | --- -/- | | |

Der vorliegende Recherchenbericht wurde für alle Patentansprüche erstellt.

| Recherchenort DEN HAAG | Abschlußdatum der Recherche 06-05-1986 | Prüfer SIMON J.J.E. |
|---|---|---|

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

EP 85 81 0419

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| E | DE-A-3 413 662 (KRATZ)<br>* Seite 8, Abschnitt 3; Seite 10, Abschnitt 1 * | 5 | |
| | --- | | |
| E | DE-A-3 422 605 (WOLFF)<br>* Seite 9, Zeile 33 - Seite 10, Zeile 22 * | 5 | |
| | ----- | | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| DEN HAAG | 06-05-1986 | SIMON J.J.E. |